# EUROPÄISCHE PATENTSCHRIFT

(11) **EP 1 263 417 B1**
(45) Veröffentlichungstag und Bekanntmachung des Hinweises auf die Patenterteilung: **02.05.2018**
(21) Anmeldenummer: 01936058.5
(22) Anmeldetag: 05.03.2001
(51) Int. Cl.: A61K 9/70

(54) **STABILISIERTE ÜBERSÄTTIGTE TRANSDERMALE THERAPEUTISCHE MATRIXSYSTEME**
STABILISED OVERSATURATED TRANSDERMAL THERAPEUTICAL MATRIX SYSTEMS
SYSTEME MATRICIEL THERAPEUTIQUE TRANSDERMIQUE SURSATURE ET STABILISE

(30) Priorität: 16.03.2000 DE 10012908
(43) Veröffentlichungstag der Anmeldung: 11.12.2002
(73) Patentinhaber: LTS LOHMANN Therapie-Systeme AG, 56626 Andernach (DE)
(72) Erfinder: MÜLLER, Walter, 56626 Andernach (DE)
(74) Vertreter: Schweitzer, Klaus
(86) Internationale Anmeldenummer: PCT/EP2001/002449
(87) Internationale Veröffentlichungsnummer: WO 2001/068060

(56) Entgegenhaltungen:
- WO-A-00/74661
- WO-A-95/18603
- WO-A-97/10812

## Beschreibung

Die Erfindung betrifft transdermale therapeutische Systeme (TTS) vom Matrix-Typ mit einer wirkstoffhaltigen Matrix auf der Basis von hydrophoben Polymeren. Insbesondere betrifft die Erfindung TTS der genannten Art, die zumindest vorübergehend mit Wirkstoff übersättigt sind, und bei denen Maßnahmen zur Verhinderung der Rekristallisation eines bei Raumtemperatur festen Wirkstoffes getroffen sind. Ferner betrifft die Erfindung Verfahren zur Herstellung von transdermalen therapeutischen Systemen der genannten Art.

Transdermale therapeutische Systeme (TTS) sind relativ neue Arzneiformen, die sich aber mittlerweile in verschiedenen Einsatzgebieten durchaus etabliert haben. Ihre allgemeinen Vorteile liegen in der Vermeidung des sogenannten "First-Pass"-Effektes und in der Aufrechterhaltung von therapeutisch sinnvollen Plasmaspiegeln über eine Zeitspanne von bis zu 7 Tagen. Jedoch sind die Einsatzmöglichkeiten von transdermalen Systemen oft dadurch eingeschränkt, daß sie sich vorwiegend für die Verabreichung sehr potenter und bereits in geringer Dosis wirksamer Arzneistoffe eignen. Dies ist durch die Barriereeigenschaften des Stratum Corneum der Haut bedingt, wodurch die Aufnahme von Arzneistoffen über die Haut eingeschränkt oder verhindert wird.

Es wurden deshalb erhebliche Anstrengungen unternommen, um dieses Hindernis zumindest teilweise zu umgehen. Dies kann beispielsweise durch den Einsatz von Permeations-Enhancern (auch Penetrations-Enhancer genannt) erreicht werden, welche die Barrierewirkung der Haut abschwächen. Ferner kann ein ausreichender Wirkstofffluß durch die Haut auch durch einen aktiven Transport des Wirkstoffes mittels elektrischem Strom erzielt werden. Eine weitere Maßnahme, durch welche die Aufnahme von Wirkstoffen über die Haut gefördert werden kann, besteht darin, daß eine möglichst hohe thermodynamische Aktivität des Wirkstoffs in dem transdermalen therapeutischen System angestrebt wird.

Permeationsenhancer sind Substanzen, die das Stratum Corneum in der Weise beeinflussen, daß der Diffusionswiderstand reduziert und so die transdermal verabreichbare Wirkstoffmenge erhöht wird. Als Permeationsenhancer eignet sich eine Vielzahl von Substanzen, beispielsweise Fettsäuren, Fettalkohole, Dimethylsulfoxid, Partialglyceride und Propylenglykol.

Transdermale Systeme, welche einen aktiven Wirkstofftransport ermöglichen, sind als sogenannte Elektrophorese- oder Iontophorese-Systeme bekannt. Derartige Systeme wurden bislang vor allem zur transdermalen Applikation von überwiegend topisch wirksamen Arzneistoffen eingesetzt. Allerdings gibt es neuere Anstrengungen, welche sich darauf konzentrieren, derartige Systeme für den praktischen Gebrauch in ihrer Größe zu minimieren, so daß sie auch für die Applikation systemisch wirksamer Arzneistoffe verwendbar sind.

Mit Ausnahme der geschilderten Elektorphorese- oder Iontophorese-Systeme beruht die Wirkstoffabgabe aus transdermalen therapeutischen Systemen grundsätzlich auf dem Prinzip der passiven Diffusion des Wirkstoffs aus dem Pflaster in und durch das Stratum Corneum der Haut, mit nachfolgender systemischer Resorption des Wirkstoffs.

Die dritte eingangs erwähnte Möglichkeit zur Verbesserung der Wirkstoffaufnahme über die Haut besteht darin, die thermodynamische Aktivität des Wirkstoffs in dem transdermalen therapeutischen System möglichst hoch zu gestalten. Auf diese Weise kann der Wirkstoffflux erhöht werden.
Eine sehr hohe thermodynamische Aktivität wird erzielt, wenn die Wirkstoffkonzentration des gelösten Wirkstoffs in den wirkstoffhaltigen Komponenten des TTS der Sättigungskonzentration des jeweiligen Wirkstoffs entspricht. Derartige TTS weisen zudem eine gute Lagerstabilität auf.

Eine weitere Steigerung der thermodynamischen Aktivität des Wirkstoffs läßt sich dadurch bewirken, daß die Konzentration des Wirkstoffs über seine Sättigungskonzentration hinaus erhöht wird. Allerdings ist der Vorteil der höheren thermodynamischen Aktivität verbunden mit dem Nachteil einer physikalischen Instabilität solcher TTS, d.h. die Lagerstabilität solcher übersättigter Systeme ist vermindert.

Der nachteilige Einfluß auf die Lagerstabilität ist darauf zurückzuführen, daß Wirkstoffe, die bei Raumtemperatur in festem Zustand vorliegen, in solchen übersättigten TTS die Tendenz haben, zu rekristallisieren. Auf Grund des Kristallwachstums bzw. der Bildung von Kristallen sinkt die Konzentration an gelöstem Wirkstoff, was zur Folge hat, daß die thermodynamische Aktivität des Wirkstoffs reduziert wird und die Abgaberate des Wirkstoffs sinkt. Aus diesem Grund ist es auch nicht möglich, übersättigte TTS mit teilweise ungelöstem Wirkstoff herzustellen, da in solchen Fällen infolge von Kristallwachstum die Konzentration des gelösten Wirkstoffs schon nach kurzer Zeit der Sättigungskonzentration entspricht.

Jedoch gibt es spezielle TTS-Formulierungen, bei denen der Zustand der Übersättigung erst nach Applikation des TTS auf die Haut eintritt, so daß die Lagerstabilität vor der Applikation nicht beeinträchtigt wird. Solche Systeme erreichen den Zustand der Übersättigung dadurch, daß ein in dem Pflaster enthaltener Lösungsvermittler ebenfalls aus dem System an die Haut abgegeben wird, bzw. dadurch, daß die Aufnahme von Feuchtigkeit aus der Haut die Sättigungslöslichkeit des Wirkstoffs im TTS vermindert. Der Vorteil solcher Systeme ist ihre Lagerstabilität bezüglich Rekristallisation. Allerdings muß auch in diesen Fällen verhindert werden, daß der Wirkstoff während der Anwendungsdauer des TTS schnell in größerem Ausmaß rekristallisiert. Dies würde dazu führen, daß während der beabsichtigten Anwendungsdauer keine ausreichende Wirkstoffabgabe erreicht werden kann.

Am einfachsten können solche während der Applikationszeit in einen übersättigten Zustand gelangenden TTS auf der Basis von Polysiloxanen hergestellt werden. Polysiloxane haben für die meisten Wirkstoffe eine nur geringe Löslichkeit. Um die Polysiloxan-Matrices derartiger TTS mit ausreichenden Mengen an gelöstem Wirkstoff beladen zu können, müssen den Polysiloxanen Lösemittel zugefügt werden. Dabei werden bevorzugt Lösemittel verwendet, die mit den Polysiloxanen nur begrenzt mischbar sind und in der Matrix in dispergierter Form als Tröpfchen vorliegen. Auf diese Weise kann eine Beeinträchtigung der physikalischen Eigenschaften der Wirkstoffmatrix weitgehend vermieden werden. Die erwähnten dispergierten Lösemittel-Tröpfchen enthalten zugleich den überwiegenden Anteil des pharmazeutischen Wirkstoffs, weshalb sie als Mikroreservoire für Wirkstoff aufgefaßt werden können.

Geeignete und physiologisch unbedenkliche Lösemittel sind z.B. Propylenglykol, 1,3-Butandiol, Dipropylenglykol, Tetrahydrofurfurylalkohol und Diethylenglykolmonoethylether. Diese Lösemittel werden ebenfalls durch die Haut resorbiert, wodurch sich der Lösemittelgehalt in der wirkstoffhaltigen TTS-Matrix verringert. Gleichzeitig konzentriert sich das von der Haut abgegebene Wasser in den Lösemittel-tröpfchen, da die Polysiloxane aufgrund ihrer extrem hydrophoben Eigenschaften nur sehr begrenzt Wasser aufnehmen können. Beide Mechanismen führen zu einer Übersättigung des Systems (TTS) mit Wirkstoff, verbunden mit einem erhöhten Wirkstoffflux durch die Haut. Zu beachten ist allerdings, daß dieser übersättigte Zustand über einen längeren Abschnitt der Applikationsdauer stabilisiert werden muß.

Die Stabilisierung des übersättigten Zustandes während des Applikationszeitraumes ist insbesondere deshalb von Bedeutung, da überraschenderweise gefunden wurde, daß bei wirkstoffübersättigten TTS mit hydrophoben Matrixformulierungen eine Rekristallisation des Wirkstoffs nicht nur in der Matrix selbst auftreten kann, sondern auch in einem dünnen Feuchtigkeitsfilm, der sich während der Applikationsdauer zwischen der wirkstoffabgebenden Seite des TTS und der darunterliegenden Hautfläche ausbilden kann.

Da der Wirkstoff nicht so schnell von der Haut aufgenommen wird, wie er aus dem TTS freigesetzt wird, ist auch dieser Feuchtigkeitsfilm mit Wirkstoff übersättigt. Folglich kann der Wirkstoff im Bereich dieses Feuchtigkeitsfilmes während des Applikationszeitraums zumindest teilweise rekristallisieren, wodurch der übersättigte Zustand aufgehoben und die thermodynamische Aktivität des Wirkstoffs herabgesetzt wird. Dies bedeutet, daß die thermodynamische Aktivität des Wirkstoffs in dem direkt über der Haut befindlichen Feuchtigkeitsfilm gegenüber den in der Matrix herrschenden Verhältnissen erniedrigt ist.
Dadurch wird die Wirkstoffaufnahme aus dem TTS durch die Haut reduziert, und die theoretischen Vorteile einer wirkstoffübersättigten Matrix gehen dabei verloren. In den polymeren Matrixschichten selbst ist die Rekristallisationstendenz, bedingt durch den verringerten Diffusionskoeffizienten und die generelle hemmende Wirkung von Polymeren auf die Bildung von Kristallkeimen, vergleichsweise schwach ausgeprägt.

Der vorliegenden Erfindung lag deshalb das Problem zugrunde, TTS vom Matrix-Typ, welche auf hydrophoben Polymeren als Matrixformulierungen basieren und zumindest während eines Teil des Anwendungszeitraums im übersättigten Zustand vorliegen, den übersättigten Zustand in der Weise zu stabilisieren, daß dieser auch während eines längeren Abschnitts der Applikationsdauer aufrechterhalten wird. Insbesondere bestand das Problem darin, zu verhindern, daß der Wirkstoff nach der Freisetzung aus dem TTS und vor seiner Aufnahme durch die Haut einer Rekristallisation unterliegt.

Überraschenderweise wurde nun gefunden, daß bei wirkstoffübersättigten, auf hydrophoben Polymeren basierenden Matrix-TTS mit den im Oberbegriff von Anspruch 1 genannten Merkmalen eine Stabilisierung des übersättigten Zustandes während der Anwendungsdauer dadurch erreicht wird, daß dem/den hydrophoben Basispolymer(en) der Wirkstoffmatrix ein Polyacrylatpolymer beigemischt ist, oder/und dadurch, daß die die hydrophoben Polymere enthaltende Matrixschicht mit einer selbstklebenden Hautkontaktschicht auf der Basis von Polyacrylaten versehen wird.

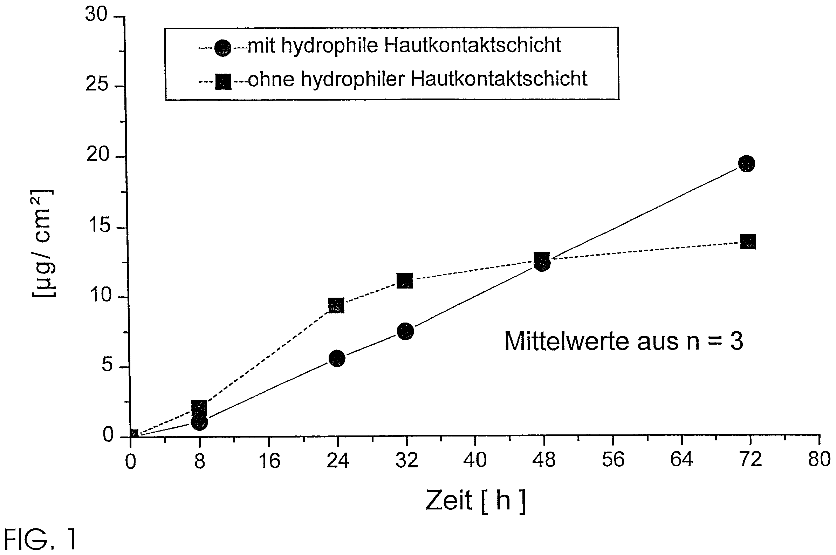

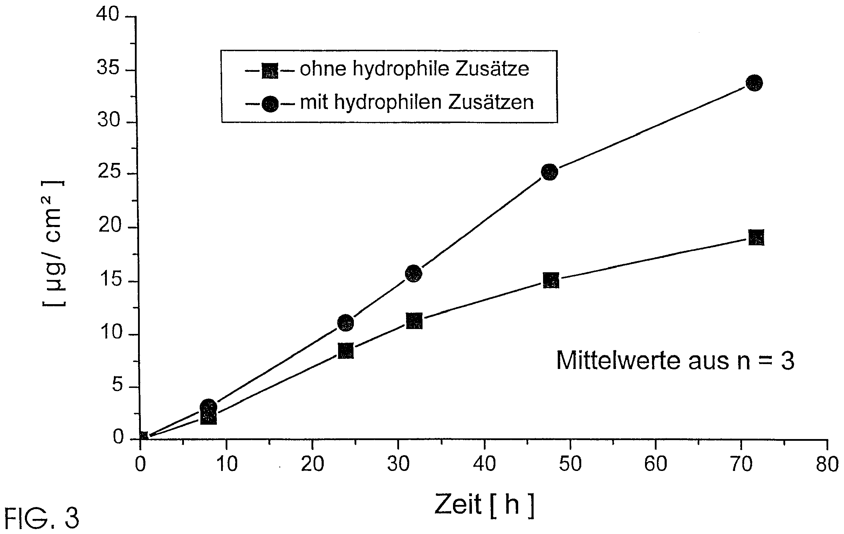

Durch die in Anspruch 1 vorgeschlagenen Maßnahmen wird die Bildung des erwähnten Feuchtigkeitsfilms verhindert oder unterdrückt und die Gefahr einer im Bereich zwischen der wirkstoffabgebenden Seite des TTS und der Haut auftretenden Wirkstoffrekristallisation reduziert oder beseitigt. Auf diese Weise bleibt die thermodynamische Aktivität des Wirkstoffs in einem solchen TTS über längere Zeit auf einem hohen Niveau (weshalb diese TTS als "stabilisiert" bezeichnet werden).
Dies hat wiederum zur Folge, daß das TTS den Wirkstoff bzw. die Wirkstoffe über einen längeren Zeitraum hinweg in therapeutischen Dosen abgeben kann, und daß auf diese Weise die Applikationszeit des TTS, während der ausreichende Abgaberaten erzielt werden müssen, verlängert wird. Insbesondere läßt sich durch die mit der Erfindung vorgeschlagenen stabilisierten TTS erreichen, daß die Wirkstoffabgabe während der Applikationszeit weitgehend konstant gehalten wird. Dies wurde durch Permeationsstudien mit den Beispielen 1 bis 3 nachgewiesen; die Ergebnisse sind in den Fig. 1 bis 3 dargestellt.

Hieraus resultieren weitere Vorteile, wie z.B. verbesserte bzw. vereinfachte Anwendung infolge der verlängerten Applikationsdauer, höhere Therapiesicherheit durch Stabilisierung des Abgabeverhaltens, sowie effizientere Wirkstoffausnutzung. Durch die Verbesserung der Wirkstoffabgabe wird durch die Erfindung ferner die Möglichkeit geschaffen, das Anwendungsspektrum von transdermalen Systemen, welche auf passiver Diffusion beruhen, zu erweitern. Des weiteren ermöglicht die Erfindung die Herstellung von transdermalen Systemen, die aufgrund der damit erzielbaren hohen Wirkstoff-Abgaberaten eine geringere Fläche aufweisen können, welches wiederum vorteilhaft bei der Herstellung und Anwendung ist.

Die vorliegende Erfindung ist anwendbar auf TTS vom Matrix-Typ (Matrix-TTS), deren wirkstoffhaltige Matrix auf der Basis von hydrophoben Polymeren hergestellt ist. Die durch zusätzliche Verwendung von Polyacrylaten erzielte stabilisierende Wirkung kommt grundsätzlich bei allen wirkstoffübersättigten hydrophoben Matrices zum Tragen.
Insbesondere ist die Erfindung bei solchen TTS von Vorteil, welche erst nach der Applikation auf die Haut durch Aufnahme von Feuchtigkeit oder durch Abgabe von Lösemitteln in einen in Bezug auf den Wirkstoff übersättigten Zustand gelangen.

Der Aufbau der erfindungsgemäßen TTS weist neben der genannten wirkstoffhaltigen Matrix eine wirkstoffundurchlässige Rückschicht und eine ablösbare, vor der Applikation zu entfernende Schutzschicht auf.
Die Erfindung betrifft TTS, welche über mehrschichtige Wirkstoffmatrices verfügen, wobei in diesem Fall nicht alle Schichten dieser Matrix klebend sein müssen. Ferner können die erfindungsgemäßen Systeme in besonderen Fällen auch eine spezielle Steuermembran enthalten, die aufgrund ihrer Dicke und/oder Zusammensetzung die Wirkstoffabgabe nach oben begrenzt.

Als hydrophobe Polymere, welche die Basispolymere für die Wirkstoffmatrix darstellen, werden bei den erfindungsgemäßen TTS vorzugsweise Polysiloxane, bevorzugt selbstklebende Polysiloxane, oder Polyisobutylen, Polyisopren oder ein Styrol-Dien-Styrol-Blockcopolymer, oder Mischungen solcher hydrophober Polymere verwendet. Unter den Polysiloxanen sind aminresistente Polysiloxane besonders bevorzugt.

Erfindungsgemäß enthalten die stabilisierten TTS ein Polyacrylat, welches der hydrophoben Matrixschicht beigemischt ist, und eine zusätzliche Hautkontaktschicht, welche auf die hydrophobe Matrixschicht aufgelagert ist und auf der Basis von Polyacrylatklebern hergestellt ist.
Bei den verwendeten Polyacrylaten handelt es sich Polymere, die - in Abhängigkeit von den eingesetzten Monomeren - mehr oder weniger hydrophile Eigenschaften aufweisen. Der Anteil des der hydrophoben Matrix beigemischten Polyacrylatpolymers beträgt liegt vorzugsweise bei höchstens 40 Gew.-%, bezogen die gesamte Matrix. Ein noch höherer Polyacrylat Anteil hätte zur Folge, dass die Eigenschaften der Wirkstoffmatrix zu stark vom Polyacrylat bestimmt werden.

Damit die erfindungsgemäße Wirkung, nämlich die Verringerung der Tendenz zur Ausbildung eines Feuchtigkeitsfilms und damit auch der Neigung zur Rekristallisation, in mindestens ausreichender Weise erzielt wird, sollte der Anteil des Polyacrylates mindestens ca. 10 Gew.-% betragen, besser noch mindestens ca. 15 Gew.-%, bezogen auf die Matrixschicht. Das beigemischte Polyacrylat kann auch ein selbstklebendes Polyacrylat sein; wenn das Polyacrylat einer selbstklebenden Matrixschicht beigemischt wird, braucht es selbst nicht selbstklebend zu sein.

Grundsätzlich ist es ausreichend, wenn zumindest der hautnahen Matrixschicht, d. h. der mit der Haut in Kontakt stehenden Matrixschicht (Hautkontaktschicht), ein hydrophiles Polyacrylatpolymer beigemischt ist. Dies trifft auf TTS mit mehrschichtigen Wirkstoffmatrices zu. Der Anteil des Polyacrylates sollte mindestens ca. 10 Gew.-% betragen, besser noch mindestens ca. 15 Gew.-%, bezogen auf die Hautkontaktschicht, jedoch höchstens 40 Gew.-%, bezogen auf die gesamte Matrix.

Neben Polyacrylaten können auch Mischungen von Polyacrylaten mit anderen hydrophilen Polymeren verwendet werden, welche erfindungsgemäß dem/den hydrophoben Basispolymeren der Matrix beigemischt werden, oder zur Herstellung einer zusätzlichen Hautkontaktschicht verwendet werden. Als weitere hydrophile Polymere können z. B. Polyvinylpyrrolidon und Copolymere des Vinylpyrrolidons mit Vinylacetat eingesetzt werden.

Auch wenn, wie vorstehend beschrieben, Mischungen von Polyacrylat (en) mit anderen hydrophilen Polymeren zum Einsatz kommen, sollte der Gesamtanteil der der hydrophoben Matrix beigemischten hydrophilen Polymere einen Wert von 40 Gew.-% nicht überschreiten, bezogen auf die gesamte Matrix.

Das erfindungsgemäß verwendete Polyacrylat selbst kann ein Copolymeres aus allen dafür geeigneten Acryl-und Methacrylderivaten und Vinylverbindungen sein. Beispielhaft werden folgende Monomere genannt: Acrylsäure, Methacrylsäure, Acrylsäureethylester, Acrylsäurebutylester, Acrylsäureoctylester, 2-Ethylhexylacrylat, 2-Hydroxyethylacrylat und Vinylacetat.

Da die die hydrophoben Polymere enthaltende Matrixschicht mit einer selbstklebenden Hautkontaktschicht auf der Basis von Polyacrylaten versehen wird, sollte die Dicke dieser Schicht oder dieses Films deutlich geringer sein als die Dicke der hydrophoben Matrixschicht (en). Insbesondere sollte die Dicke der genannten Schicht einen Wert nicht überschreiten, der 50 % der Dicke der hydrophoben Matrixschicht (en) entspricht, da sonst die Eigenschaften der zusätzlichen hydrophilen Hautkontaktschicht - die auch wirkstoffhaltig ist - die Eigenschaften des Systems dominieren würde.

Gemäß einer besonderen Ausführungsform der Erfindung ist vorgesehen, dass die selbstklebende, hydrophile Hautkontaktschicht eine Mischung aus einem selbstklebenden Polyacrylat und einem hydrophilen Polymer, vorzugsweise einem filmbildenden Polymer, darstellt. Als filmbildendes hydrophiles Polymer kann beispielsweise Polyvinylpyrrolidon oder ein Copolymeres von Vinylpyrrolidon und Vinylacetat verwendet werden.

Die Herstellung von erfindungsgemäßen stabilisierten TTS kann in der Weise erfolgen, dass zunächst eine Lösung hergestellt wird, welche die hydrophoben Basispolymere und die beigemischten Acrylatpolymere, sowie ggf. Hilfsstoffe, in einem geeigneten Lösemittel enthält. Zu dieser Matrixpolymerlösung wird der Wirkstoff hinzugegeben und gelöst. Falls erforderlich, kann der Wirkstoff auch in gelöster Form zugesetzt werden, eventuell unter Verwendung speziell für diesen Wirkstoff geeigneter Lösemittel. Die erhaltene wirkstoffhaltige Matrixpolymermasse wird sodann auf eine geeignete Folie beschichtet und einer Trocknung oder Wärmebehandlung unterzogen, um die Lösemittel der Polymere zu entfernen.

Bei der Herstellung von erfindungsgemäßen stabilisierten TTS, welche durch eine selbstklebende hydrophile Hautkontaktschicht auf der Basis von Polyacrylaten gekennzeichnet sind, werden die hydrophobe, wirkstoffhaltige Matrixschicht und die hydrophile Hautkontaktschicht in getrennten Beschichtungsvorgängen hergestellt. Anschließend werden die einzelnen Schichten aufeinanderlaminiert, wodurch das vollständige System erhalten wird.

Die Hautkontaktschicht kann dabei schon während der Herstellung mit Wirkstoff beladen werden, oder sie kann wirkstofffrei hergestellt werden. Im letztgenannten Fall gelangt der Wirkstoff nach Herstellung des Laminats durch Diffusion aus der hydrophoben Matrixschicht in die Hautkontaktschicht.

Die Erfindung wird nachfolgend an Hand von Beispielen erläutert.

### Beispiel 1

### TTS mit Estradiol

### Beispiel 1a:

### TTS ohne hydrophile Hautkontaktschicht

### (Vergleichsbeispiel 1)

1,0 g Estradiol-hemihydrat wurden in 22,75 g 1,3-Butandiol gelöst und die Lösung durch Zugabe von 0,7 g Hydroxypropylcellulose verdickt. Zu der Lösung werden nun 60 g einer Lösung eines aminresistenten Polysiloxanklebers (BIO-PSA 4301; Dow-Corning; Feststoffgehalt: 70 Gew.-%) gegeben und die Wirkstofflösung in der Lösung des Klebers durch Rühren dispergiert.
Anschließend wird die Masse mit einem Erichson-Rakel auf eine abhäsiv ausgerüstete Folie (Scotchpak 1022; 3M) in einer Dicke von 200 µm beschichtet und bei 40 °C 20 min getrocknet. Es resultiert ein Matrixfilm mit einem Beschichtungsgewicht von 120 g/m². Der getrocknete Matrixfilm wird mit der Rückschicht des TTS (Scotchpak 1220; 3M) abgedeckt.

### Beispiel 1b:

### TTS mit hydrophiler Hautkontaktschicht

1,0 g Estradiol-hemihydrat werden in 20,0 g 1,3-Butandiol gelöst und anschließend 20,0 g einer Kollidon 90F-Lösung (Kollidon 90F ist ein Polyvinylpyrrolidon; BASF) mit einem Feststoffgehalt von 25 Gew.-% unter Rühren zugegeben. Danach werden 145 g einer Lösung eines Polyacrylatklebers (Durotak 387-2287; National Starch & Chemical; Feststoffgehalt: 51 Gew.-%) zugegeben und die Mischung durch Rühren homogenisiert. Die Masse wird mit einem Erichson-Rakel in einer Dicke von 50 µm auf eine abhäsiv ausgerüstete Folie (Scotchpak 1022; 3M) beschichtet und bei 40 °C 15 min getrocknet.

Der getrocknete Film hat ein Beschichtungsgewicht von 16 g/m².

Von der unter 1a hergestellten hydrophoben Matrixschicht wird nun die abhäsiv ausgerüstete Folie entfernt und die Matrixschicht auf die Hautkontaktschicht auflaminiert.
Aus diesem Gesamtlaminat werden nun die fertigen TTS ausgestanzt.

Die Ergebnisse einer vergleichenden Permeationsstudie zwischen Muster ohne Hautkontaktschicht (1a) und Muster mit hydrophiler Hautkontaktschicht (1b) sind in Fig. 1 dargestellt.

### Beispiel 2:

### Transdermales System (TTS) mit Estradiol

### Beispiel 2a:

### TTS ohne hydrophile Hautkontaktschicht

### (Vergleichsbeispiel 2)

5,0 g Estradiol-hemihydrat werden in 38,5 g Dipropylenglykol gelöst. Zu dieser Lösung werden 124 g einer Lösung eines Polysiloxanklebers (BIO-PSA 4301; Dow-Corning; Feststoffgehalt: 70 Gew.-%) gegeben und die Wirkstofflösung in der Kleberlösung unter Rühren dispergiert.
Danach wird die Masse mittels eines Erichson-Rakels auf eine geeignete abhäsive Folie (Scotchpak 1022; 3M) beschichtet und das Lösemittel des Klebers durch 20minütiges Trocknen bei 45 °C entfernt. Der getrocknete Film mit einem Beschichtungsgewicht von 80 g/m² wird dann mit einer geeigneten Folie (z. B. Scotchpak 1220; 3M) abgedeckt.

### Beispiel 2b:

### TTS mit hydrophiler Hautkontaktschicht

1,0 g Estradiol-hemihydrat werden in 10,0 g Dipropylenglykol gelöst und anschließend 20,0 g einer Kollidon 90F Lösung (Kollidon 90F ist ein Polyvinylpyrrolidon) mit einem Feststoffgehalt von 25 Gew.-% unter Rühren zugegeben.
Danach werden 164 g einer Lösung eines Polyacrylatklebers (Durotak 387-2287 ; National Starch & Chemical ; Feststoffgehalt : 51 Gew.-%) zugegeben und die Mischung durch Rühren homogenisiert. Die Masse wird mit einem Erichson-Rakel in einer Dicke von 50 um auf eine abhäsiv ausgerüstete Folie (Scotchpak 1022 ; 3M) beschichtet und bei 40 C 15 min getrocknet. Der getrocknete Film hat ein Beschichtungsgewicht von 15 g/m2.

Von der unter 2a hergestellten hydrophoben Matrixschicht wird die abhäsiv ausgerüstete Schutzfolie entfernt und die Matrixschicht auf die Hautkontaktschicht auflaminiert.
Aus diesem Gesamtlaminat werden nun die fertigen TTS ausgestanzt.

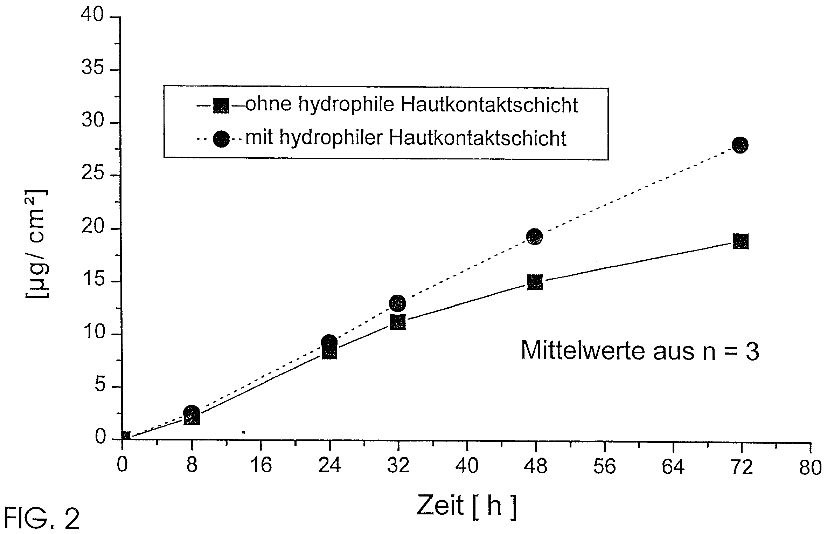

Die Ergebnisse einer vergleichenden Permeationsstudie zwischen Mustern ohne Hautkontaktschicht (2a) und Muster mit hydrophiler Hautkontaktschicht (2b) sind in Fig. 2 dargestellt.

Permeationsstudien mit den gemäß Beispiel 1 und 2 gefertigten Systemen.

Die Ergebnisse der Vergleichsmessungen sind in Fig. 1 und 2 dargestellt. Sie wurden durchgeführt unter Verwendung von Franz-Diffusionszellen und menschlicher Epidermis. Jeder Punkt ist das Mittel aus 3 unabhängigen Messungen.

Der zeitliche Verlauf der Hautpermeation in den Fig. 1 und 2 lässt deutlich erkennen, dass bei den erfindungsgemäßen TTS über einen Zeitraum von mindestens 72 h eine gleichbleibende Abgaberate und somit eine Stabilisierung erzielt wird, während bei den Vergleichsbeispielen bereits nach 32 h eine deutliche Abflachung des Permeationsprofils zu erkennen ist.

## Patentansprüche

1. Transdermales therapeutisches System vom Matrix-Typ, mit einer wirkstoffundurchlässigen Rückschicht, einer ablösbaren Schutzschicht und einer wirkstoffhaltigen Matrix auf der Basis von hydrophoben Polymeren, wobei der Wirkstoff einen Schmelzpunkt oberhalb der Raumtemperatur hat, und das System erst nach der Applikation auf die Haut in einen in Bezug auf den Wirkstoff übersättigten Zustand gelangt, **dadurch gekennzeichnet, dass** die wirkstoffhaltige Matrix eine einschichtige oder mehrschichtige Matrix auf der Basis von Polysiloxanen ist und einen Zusatz mindestens eines für den Wirkstoff geeigneten Lösungsmittels enthält, die mit den Polysiloxanen nur begrenzt mischbar sind und in der Matrix in dispergierter Form als Tröpfchen vorliegen, und
die genannte wirkstoffhaltige Matrix mit einer selbstklebenden, wirkstoffhaltigen Hautkontaktschicht auf Basis von hydrophilen Polyacrylaten versehen ist.

2. Transdermales therapeutisches System nach Anspruch 1, **dadurch gekennzeichnet, dass** es sich bei den Polysiloxanen um aminresistente Polysiloxane handelt.

3. Transdermales therapeutisches System nach Anspruch 1, **dadurch gekennzeichnet, dass** es sich bei den Polysiloxanen um selbstklebende Polysiloxane handelt.

4. Transdermales therapeutisches System vom Matrix-Typ nach einem der Ansprüche 1 bis 3, **dadurch gekennzeichnet, dass** die Hautkontaktschicht aus einer Mischung eines selbstklebenden Polyacrylats und eines hydrophilen Polymers, vorzugsweise eines filmbildenden hydrophilen Polymers, besteht.

5. Transdermales therapeutisches System vom Matrix-Typ nach Anspruch 4, **dadurch gekennzeichnet, dass** das filmbildende hydrophile Polymer Polyvinylpyrrolidon oder ein
Copolymeres von Vinylpyrrolidon und Vinylacetat ist.

6. Verfahren zur Herstellung eines transdermalen therapeutischen Systems nach einem der Ansprüche 1 bis 5, welches folgende Schritte aufweist:
a) Herstellung einer Lösung oder Dispersion, enthaltend ein Polysiloxan als hydrophobes Basispolymer und einen Wirkstoff sowie mindestens ein für den Wirkstoff geeignetes Lösungsmittel, das mit dem Polysiloxan nur begrenzt mischbar ist und in der Matrix in dispergierter Form als Tröpfchen vorliegt;
b) Beschichten dieser polymer- und wirkstoffhaltigen Masse in dünner Schicht auf eine abhäsiv ausgerüstete Folie, anschließend Trocknung;
c) Herstellung einer zweiten Schicht - der Hautkontaktschicht - durch Beschichten und anschließendem Trocknen einer Beschichtungsmasse, die ein Acrylatpolymer, gegebenenfalls den Wirkstoff und gegebenenfalls zusätzlich ein hydrophiles filmbildendes Polymer enthält, oder ein hydrophobes Polymer abgemischt mit einem Acrylatpolymer und gegebenenfalls zusätzlich mit einem hydrophilen filmbildenden Polymer;
d) Ablösen der abhäsiven Folie von der in Schritt b) erhaltenen Matrixschicht;
e) Auflaminieren der in Schritt b) erhaltenen Matrixschicht auf die in Schritt c) erhaltene Hautkontaktschicht;
f) Ausstanzen einzelner transdermaler therapeutischer Systeme.

## Claims

1. Transdermal matrix-type therapeutic system comprising an active-ingredient-impermeable backing layer, a removable protective layer and an active-ingredient-containing matrix based on hydrophobic polymers, wherein the active ingredient has a melting point above room temperature and the system gets into a supersaturated state with respect to the active ingredient only after application to the skin, **characterized in that** the active-ingredient-containing matrix is a monolayer or multilayer matrix based on polysiloxanes and contains an addition of at least one solvent suitable for the active ingredient, which solvents are miscible with the polysiloxanes only to a limited extent and are present in the matrix in dispersed form as droplets, and
the stated active-ingredient-containing matrix is provided with a self-adhesive, active-ingredient-containing skin contact layer based on hydrophilic polyacrylates.

2. Transdermal therapeutic system according to Claim 1, **characterized in that** the polysiloxanes are amine-resistant polysiloxanes.

3. Transdermal therapeutic system according to Claim 1, **characterized in that** the polysiloxanes are self-adhesive polysiloxanes.

4. Transdermal matrix-type therapeutic system according to any of Claims 1 to 3, **characterized in that** the skin contact layer consists of a mixture of a self-adhesive polyacrylate and a hydrophilic polymer, preferably a film-forming hydrophilic polymer.

5. Transdermal matrix-type therapeutic system according to Claim 4, **characterized in that** the film-forming hydrophilic polymer is polyvinylpyrrolidone or a copolymer of vinylpyrrolidone and vinyl acetate.

6. Method for preparing a transdermal therapeutic system according to any of Claims 1 to 5, comprising the following steps:
a) preparing a solution or dispersion containing a polysiloxane as hydrophobic base polymer and an active ingredient and also at least one solvent suitable for the active ingredient, which solvent is miscible with the polysiloxane only to a limited extent and is present in the matrix in dispersed form as droplets;
b) coating said polymer-containing and active-ingredient-containing composition in a thin layer onto an abhesively finished film, followed by drying;
c) preparing a second layer - the skin contact layer - by coating and subsequently drying a coating composition which contains an acrylate polymer, optionally the active ingredient and optionally additionally a hydrophilic film-forming polymer, or a hydrophobic polymer mixed with an acrylate polymer and optionally additionally with a hydrophilic film-forming polymer;
d) removing the abhesive film from the matrix layer obtained in step b);
e) laminating the matrix layer obtained in step b) onto the skin contact layer obtained in step c);
f) punching out individual transdermal therapeutic systems.

## Revendications

1. Système thérapeutique transdermique du type matrice, avec une couche arrière imperméable au principe actif, une couche de protection amovible et une matrice contenant le principe actif à base de polymères hydrophobes, dans lequel le principe actif a un point de fusion supérieur à la température ambiante, et le système ne parvient à un état sursaturé par rapport au principe actif qu'après l'application sur la peau, **caractérisé en ce que** la matrice contenant le principe actif est une matrice monocouche ou multicouche à base de polysiloxanes et contient un additif d'au moins un solvant convenant au principe actif, qui peuvent être mélangés seulement de manière limitée avec les polysiloxanes et qui sont présents dans la matrice sous forme dispersée en tant que gouttelettes, et
ladite matrice contenant le principe actif est dotée d'une couche venant au contact de la peau contenant le principe actif, auto-adhésive, à base de polyacrylates hydrophiles.

2. Système thérapeutique transdermique selon la revendication 1, **caractérisé en ce que** les polysiloxanes sont des polysiloxanes résistants aux amines.

3. Système thérapeutique transdermique selon la revendication 1, **caractérisé en ce que** les polysiloxanes sont des polysiloxanes auto-adhésifs.

4. Système thérapeutique transdermique du type matrice selon l'une quelconque des revendications 1 à 3, **caractérisé en ce que** la couche venant au contact de la peau se compose d'un mélange d'un polyacrylate auto-adhésif et d'un polymère hydrophile, de préférence d'un polymère hydrophile filmogène.

5. Système thérapeutique transdermique du type matrice selon la revendication 4, **caractérisé en ce que** le polymère hydrophile filmogène est la polyvinylpyrrolidone ou un copolymère de vinylpyrrolidone et d'acétate de vinyle.

6. Procédé de fabrication d'un système thérapeutique transdermique selon l'une quelconque des revendications 1 à 5, qui comprend les étapes suivantes :
a) fabrication d'une solution ou dispersion, contenant un polysiloxane en tant que polymère de base hydrophobe et un principe actif ainsi qu'au moins un solvant convenant au principe actif, qui peut être mélangé seulement de manière limitée avec le polysiloxane et qui est présent dans la matrice sous forme dispersée en tant que gouttelettes ;
b) revêtement de cette masse contenant le polymère et le principe actif en fine couche sur un film réalisé de manière non adhésive, puis séchage ;
c) fabrication d'une deuxième couche - la couche venant au contact de la peau - par revêtement puis séchage d'une masse de revêtement, qui contient un polymère d'acrylate, le cas échéant le principe actif et le cas échéant en plus un polymère filmogène hydrophile, ou un polymère hydrophobe mélangé avec un polymère d'acrylate ou le cas échéant en plus avec un polymère filmogène hydrophile ;
d) décollement du film non adhésif de la couche matricielle obtenue à l'étape b) ;
e) stratification de la couche matricielle obtenue à l'étape b) sur la couche venant au contact de la peau obtenue à l'étape c) ;
f) découpage de systèmes thérapeutiques transdermiques individuels.
